(19) Europäisches Patentamt
European Patent Office
Office européen des brevets

(11) **EP 2 756 249 B1**

(12) **EUROPÄISCHE PATENTSCHRIFT**

(45) Veröffentlichungstag und Bekanntmachung des Hinweises auf die Patenterteilung:
**16.09.2015 Patentblatt 2015/38**

(21) Anmeldenummer: **12745635.8**

(22) Anmeldetag: **07.08.2012**

(51) Int Cl.:
*F27D 17/00* ^(2006.01)   *C07C 29/151* ^(2006.01)

(86) Internationale Anmeldenummer:
**PCT/EP2012/003371**

(87) Internationale Veröffentlichungsnummer:
**WO 2013/037444 (21.03.2013 Gazette 2013/12)**

(54) **VERFAHREN ZUR GEWINNUNG VON OLEFINEN AUS OFENGASEN VON STAHLWERKEN**

METHOD FOR OBTAINING OLEFINS FROM FURNACE GASES OF STEEL WORKS

PROCÉDÉ DE RÉCUPÉRATION D'OLÉFINES À PARTIR DE GAZ DE FOURNEAUX D'ACIÉRIES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.09.2011 DE 102011113547**
**17.11.2011 EP 11009118**

(43) Veröffentlichungstag der Anmeldung:
**23.07.2014 Patentblatt 2014/30**

(73) Patentinhaber: **Linde Aktiengesellschaft 80331 München (DE)**

(72) Erfinder:
• **SCHÖDEL, Nicole**
**81477 München (DE)**
• **HAIDEGGER, Ernst**
**85521 Riemerling (DE)**
• **SCHMIGALLE, Holger**
**(verstorben) (DE)**
• **GÖKE, Volker**
**82538 Geretsried (DE)**
• **SCHMADERER, Harald**
**82515 Wolfratshausen (DE)**

(56) Entgegenhaltungen:
**CN-A- 101 823 937    US-A1- 2011 112 314**

## EP 2 756 249 B1

**Beschreibung**

Die Erfindung betrifft ein Verfahren zur Verarbeitung von Abgas aus einem Stahlwerk und/oder einem Hüttenwerk, wobei das Abgas Kohlenmonoxid und/oder Kohlendioxid enthält.

In einem Hüttenwerk wird mit einem Hochofen aus Eisenerz durch Reduktion Eisen gewonnen. Als Reduktionsmittel wird dabei im Wesentlichen Koks verwendet. Der Hochofen ist in Schachtbauweise gebaut und wird von oben abwechselnd mit einer sogenannten Möllerschicht (ein Gemisch aus Eisenerz und Zusatzstoffen) und einer Koksschicht beschickt. Im Hochofen herrschen Temperaturen zwischen 2000 °C und 200° C wobei die Temperatur von unten nach oben abnimmt.

Der im Wesentlichen aus Kohlenstoff bestehende Koks reagiert mit Sauerstoff stark exotherm zu Kohlendioxid und erzeugt so am Boden des Hochofens Temperaturen zwischen 1800°C und 2000 °C, bei der Verwendung von reinem Sauerstoff bis zu 2200°C. Unmittelbar an die exotherme Reaktion schließen sich die beiden endothermen Reaktionen unter Bildung von Kohlenmonoxid an:

$$CO_2 + C \dashrightarrow 2\ CO$$

$$H_2O + \dashrightarrow CO + H_2$$

Kohlenmonoxid und Wasserstoff dienen im Hochofen als Reduktionsmittel und reduzieren die Eisenoxide des Eisenerzes zu Eisen ebenso wie die Oxide der Eisenbegleiter Mangan, Silizium und Phosphor. Dabei nimmt das Eisen allerdings einen Teil Kohlenstoff auf.

Das mit Kohlenstoff beladen Eisen wird vom Boden des Hochofens als Roheisen gewonnen. Am oberen Ende des Hochofens (Kopf) sammelt sich entsprechend ein Abgas welches auch als Gichtgas oder Hochofengas bezeichnet wird. Dieses Abgas besteht im Wesentlichen aus Kohlenoxiden (Kohlenmonoxid, Kohlendioxid zu je 15-25 Vol-%, Stickstoff (50-60 Vol-%) sowie 3 Vol-% Wasserstoff, 0.5-1 Vol-% Methan und Wasser) neben weiteren Spurenelementen. Ein derartiges Abgas soll mit dem erfindungsgemäßen Verfahren behandelt werden.

Das im Hochofen entstehende Roheisen ist aufgrund des Kohlenstoffanteils nicht schmiedbar und wird in einem Stahlwerk weiterverarbeitet. Dort wird hauptsächlich in einem Konverterofen der Kohlenstoff des Eisens abgebrannt. Dieser Prozess wird auch als Frischen bezeichnet. Zumeist wird im Konverterofen über eine oder mehrere geeignete Düsenlanzen reiner Sauerstoff auf und/oder in das heiße Roheisen geblasen. Der Kohlenstoff wird dabei zu Kohlendioxid oxidiert und es entsteht durch die hohen Temperaturen flüssiger Stahl. Auch im Konverterofen entsteht somit ein Abgas, welches hauptsächlich aus Kohlendioxid (ca. 20 Vol-%) und Kohlenmonoxid (ca. 55 Vol-%) nebst Wasserstoff (ca. 4 Vol-%) besteht und dessen Behandlung ebenfalls Gegenstand der vorliegenden Erfindung ist.

Häufig sind Hüttenwerk und Stahlwerk in einer Anlage vereint und als Stahlwerk bezeichnet. In diesem Fall können gemäß der vorliegenden Erfindung sowohl das Abgas des Hochofens als auch das Abgas des Konverterofens oder beide gemeinsam behandelt werden.

In den meisten Fällen ist in derartigen Anlagen auch eine Kokerei integriert, bei der in einem Koksofen aus Kohle Koks erzeugt wird. Der im Hochofen benötigte Koks wird in sogenannten Kokereien hergestellt, welche häufig in die Hüttenwerke oder Stahlwerke integriert sind. Dabei werden in einem Koksofen die flüchtigen Bestandteile in der Kohle durch das Erhitzen auf eine Temperatur von 900 °C und 1400 °C pyrolysiert, freigesetzt und abgesaugt. Dabei entsteht der im Wesentlichen aus Kohlenstoff bestehende Koks und ein als Kokereigas bezeichnetes Abgas, welches die flüchtigen Bestandteile enthält. Die Pyrolyse im Koksofen findet in Abwesenheit von Sauerstoff statt. Das entstandene Kokereigas enthält Wasserstoff (ca. 55%), Methan, Stickstoff, Kohlenmonoxid, Kohlendioxid, Schwefel und höhere Kohlenwasserstoffe.

Die beiden vorgenannten Anlagentypen sind hier nur beispielhaft erwähnt. Die Erfindung ist daher nicht auf diese Anlagentypen beschränkt. Generell ist die Erfindung auf die Behandlung von Abgasen aus Hütten- und/oder Stahlwerken gerichtet, die Kohlendioxid und/oder Kohlenmonoxid aufweisen. D.h. auch die Abgase von Stahlwerken oder Hüttenwerken bei denen die Reduktion des Eisenerzes mittels Lichtbogenöfen, Direktreduktion oder ähnliches Verfahren erzeugt, werden von der vorliegenden Erfindung erfasst, so sie denn Kohlenmonoxid und/oder Kohlendioxid enthalten. Generell dient die Erfindung der Behandlung von Abgasen metallurgischer Öfen wie beispielsweise dem Konverterofen.

Ein Verfahren zur Behandlung eines derartigen Abgases wird beispielsweise in der US-Schrift US2011/0041517 beschrieben. Gemäß dem hier offenbarten Stand der Technik wird das heiße Abgas eines metallurgischen Ofens durch Zugabe eines Reduktionsmittels reformiert, wobei das Reduktionsmittel zugegeben wird, wenn die Sauerstoffkonzentration 1 Vol-% oder weniger beträgt und die Reformierung als vollständig angesehen wird, wenn die Temperatur des Abgases 800 °C oder höher beträgt. Dabei soll das heiße Abgas gekühlt werden, die Kohlendioxid Emission in die Umwelt minimiert und das Abgas einen höheren Brennwert erhalten, da derartige Abgase nach dem Stand der Technik in eine Gasturbine zur Erzeugung elektrischer Energie geführt werden.

WO 2009/023987, CN 101 913 558, CN 101 823 937 und CN 102 079 689 offenbaren Verfahren zum Herstellen von Methanol beziehungsweise Dimethylether aus Kokereiabgas und aus bei der Stahlherstellung anfallendem Abgas.

Die US 2011/0112314 beinhaltet ein Verfahren zur Herstellung von Olefinen aus sauerstoffhaltigen Einsatzprodukten.

Ebenfalls ist bekannt, gasförmige oder flüssige Kohlenwasserstoffe mit Hochtemperaturabgasen zu mischen. Derartige Hochtemperaturabgase enthalten Kohlendioxid und/oder Wasserdampf und stammen vor allem aus dem Konverterofen. Durch die Zumischung der Kohlenwasserstoffe wird in einer Reformierungsreaktion unter Wärmeverbrauch der Kohlenmonoxid- und der Wasserstoffanteil erhöht und somit der Brennwert des Abgases gesteigert.

Der vorliegenden Erfindung liegt die Aufgabe zugrunde, eine alternative Verarbeitung eines Abgases der eingangs erwähnten Art zu entwickeln. Eine weitere Aufgabe der vorliegenden Erfindung ist die Gewinnung von Wertprodukten aus derartigen Abgasen. In derartigen Abgasen vorhandene Treibhausgase wie Kohlendioxid, Kohlenmonoxid und Methan sollen nicht in die Atmosphäre gelangen sondern möglichst vollständig in Wertprodukte umgewandelt werden.

Diese Aufgabe wird durch die Merkmalskombination des unabhängigen Anspruches 1 gelöst. Weitere vorteilhafte Ausgestaltungen der Erfindung werden in den Unteransprüchen gegeben.

Bei dem erfindungsgemäßen Verfahren zur Verarbeitung von Abgas aus einem Stahlwerk und/oder einem Hüttenwerk, wobei das Abgas Kohlendioxid und/oder Kohlenmonoxid enthält, wird das Abgas zumindest teilweise zusammen mit einem wasserstoffhaltigen Gas in ein Verfahren zur Bildung von Dimethylether geführt, wodurch ein DME-haltiges Produktgas gebildet wird.

Erfindungsgemäß wird das DME-haltige Produktgas in ein Verfahren zur Umwandlung von Diemethylether zu Olefinen geführt, wodurch ein olefinhaltiges Produktgas gebildet wird, und wobei mittels Trennverfahren Olefine, insbesondere Ethylen und/oder Propylen, aus dem olefinhaltigen Produktgas abgetrennt wird/werden.

Weiterhin wird erfindungsgemäß am Eingang des Verfahrens zur Bildung von Dimethylether ein Verhältnis von Wasserstoff zu Kohlenmonoxid gewichtet mit der Kohlendioxidkonzentration $\frac{c[H2] - c[CO2]}{c[CO] + c[CO2]}$ von 0.9 bis 1.1 eingestellt und Dimethylether gebildet. Vorteilhafterweise wird dabei auch Kohlendioxid aus Kohlenmonoxid gebildet.

Dabei der Wasserstoffgehalt derart reguliert, dass die Reaktion selektiv für Dimethylether abläuft, je nach dem weiteren konkreten Verfahren (Katalysator etc.) zur Bildung der Olefine, insbesondere Ethylen.

Das erfindungsgemäße olefinhaltige Produktgas kann dabei entweder in den Zerlegungsteil einer bestehenden Olefinanlage eingespeist werden, wo Ethylen und/oder Propylen ebenfalls als Wertprodukte abgetrennt werden, oder es werden die Hauptwertprodukte Ethylen und/oder Propylen in einer separaten Trennanlage aus dem olefinhaltigen Produktgas gewonnen.

Der Grundgedanke der Erfindung besteht darin, die Abgase nicht ausschließlich als Einsatzstoffe für Verfahren zur Erzeugung elektrischer Energie beispielsweise mittels Gasturbine zu betrachten. Gemäß der Erfindung werden Kohlendioxid, Kohlenmonoxid und/oder Methan im Abgas nicht nur in Dimethylether zur Erhöhung des Brennwertes umgewandelt. Das gewonnene DME-haltige Produktgas wird als Einsatzstoff in ein Verfahren zur Erzeugung von Olefinen geführt, so dass ein olefinhaltiger Produktstrom gewonnen wird. Aus diesem Produktstrom werden die Olefine, insbesondere Ethylen und Propylen, mittels bekannter Trennverfahren abgetrennt und als Wertprodukt/e gewonnen. Zumindest einem Teil des Abgases wird dabei vor dem Verfahren zur Erzeugung von Dimethylether ein wasserstoffhaltiges Gas zugemischt, um sicher zu stellen, dass der Wasserstoffanteil im Einsatz des Verfahrens zur Erzeugung von Dimethylether hinreichend hoch ist, um nahezu alle Kohlenoxide zu Diemethylether umzuwandeln und einen hohen Anteil der beiden Stoffe im DME-haltigen Produktgas zu erhalten.

Prinzipiell wird durch das erfindungsgemäße Verfahren durch die Mischung von zumindest einem Teil des Abgases mit einem wasserstoffhaltigen Gas eine Art Synthesegas aus Kohlenmonoxid und Wasserstoff erzeugt. Dabei wird erfindungsgemäß zumindest ein Teil des Abgases mit dem wasserstoffhaltigen Gas gemischt. Die mit dem wasserstoffhaltigen Gas zu vermischende Menge des Abgases hängt dabei von den wirtschaftlichen Gegebenheiten am jeweiligen Standort ab. Bei einer hinreichend hohen Menge an günstigen und verfügbaren Wasserstoff kann das anfallende Abgas komplett mit dem wasserstoffhaltigen Gas vermischt werden, wobei soviel wasserstoffhaltiges Gas zugemischt werden kann, dass auch im Abgas vorhandenes Kohlendioxid möglichst vollständig in Kohlenmonoxid umgewandelt wird. Ist nur eine geringe Menge günstiger Wasserstoff verfügbar, wird dieser möglichst komplett genutzt und nur ein entsprechender Teil des Abgases mit dem wasserstoffhaltigen Gas gemischt.

Verfahren zur Umwandlung von beispielsweise Methanol in Olefine (z.B. Erzeugung von Ethylen durch katalytische Dehydrierung von Methanol über Aluminium und Zeolith Katalysatoren) sind im Stand der Technik bekannt und werden beispielsweise in "Ethylene", H. Zimmermann und R. Walzl in Ullmann's Encyclopedia of Industrial Chemistry, Wiley 2011 beschrieben. Gleiches gilt für die Abtrennung von Olefinen, insbesondere Ethylen und Propylen, aus derartigen olefinhaltigen Strömen (siehe gleiche Referenz und darin enthaltene Referenzen). Die vorliegende Erfindung ist jedoch nicht auf die dort beschriebenen Verfahren und die dort beschriebenen Trennverfahren limitiert.

Grundsätzlich kombiniert die Erfindung Verfahren zur Stahlherstellung und Verfahren zur Gewinnung von Olefinen, insbesondere Ethylen. Gemäß der Erfindung werden alle geeigneten Bestandteile der Abgase aus Stahl- und/oder Hüttenwerken mit Wasserstoff in Dimethylether umgewandelt und in einem weiteren Schritt aus Dimethylether Olefine, insbesondere Ethylen, gebildet, welches dann als Wertprodukt aus dem Abgas abgetrennt wird. So werden durch die vorliegende Erfindung aus den Abgasen Wertprodukte gewonnen und die Abgase nicht nur wie im Stand der Technik bezüglich des Brennwertes optimiert und zur Erzeugung elektrischer Energie verwendet.

Bevorzugt wird das Abgas aus einem Hochofen und/oder einem Konverterofen und /oder aus einem Direktreduktionsverfahren für Eisenerz abgeführt. Wie bereits eingangs erwähnt enthält das Gichtgas genannte Abgas von Hochöfen ebenso wie das Abgas von Konverteröfen hauptsächlich Kohlendioxid und Kohlenmonoxid, wodurch beide Abgase für die vorliegende Erfindung geeignet sind. Die Abgase können dabei getrennt oder bevorzugt gemeinsam mit dem erfindungsgemäßen Verfahren behandelt werden.

Insbesonders Abgase aus einem Direktreduktionsverfahren für Eisenerz sind für das erfindungsgemäße Verfahren geeignet. Abgase aus dem Direktreduktionsverfahren für Eisenerz enthalten Kohelnmonoxid und Wasserstoff in einem Verhältnis zueinander, das zur Herstellung von Dimethylether ganz besonders geeignet ist.

Mit besonderem Vorteil wird Kokereiabgas als wasserstoffhaltiges Gas zusammen mit dem Abgas in das Verfahren zur Bildung von Dimethylether geführt. Diese Ausgestaltung der Erfindung eignet sich besonders für sogenannte integrierte Hüttenwerke oder Stahlwerke. Wie bereits erwähnt wird in einer Kokerei Koks aus Kohle erzeugt. Das im Koksofen dabei entstehende Kokereigas enthält Wasserstoff (ca. 55 Vol-%), Methan (ca. 20 Vol-%), Stickstoff, Kohlenmonoxid, Kohlendioxid, Schwefel enthaltende Verbindungen und höhere Kohlenwasserstoffe. Daher eignet sich das Kokereigas besonders als wasserstoffhaltiges Gas, da es zum einen in den integrierten Hüttenwerken oder Stahlwerken vorhanden ist und zusätzlich noch Kohlendioxid, Kohlenmonoxid und Methan enthält, welche katalytisch Dimethylether umgewandelt werden können.

In einer Ausgestaltung der Erfindung werden das Kohlenmonoxid und/oder Kohlendioxid enthaltende Abgas und/oder das wasserstoffhaltige Gas aufgereinigt, bevor beide als Einsatz in das Verfahren zur Bildung von Dimethylether geführt werden. Dabei können beispielsweise aus dem Abgas alle Bestandteile bis auf Kohlenmonoxid und/oder Kohlendioxid entfernt werden. Das wasserstoffhaltige Gas besteht nach der Aufreinigung zweckmäßigerweise nur noch aus Wasserstoff und optional Kohlenmonoxid und/oder Kohlendioxid.

Vorteilhafterweise das olefinhaltige Produktgas nach Abtrennung der Olefine als alkanhaltiges Restgas zur Unterfeuerung in den Koksofen und/oder Hochofen zurückgeführt wird. In den Abgasen der Öfen sind zum einen in geringem Anteil Kohlenwasserstoffe (vor allem Alkane) vorhanden, zum anderen werden in Nebenreaktionen bei der Olefinbildung auch Alkane gebildet. Nach Abtrennung der Olefine, insbesondere Ethylen und/oder Propylen, aus dem olefinhaltigen Produktgas besteht das alkanhaltige Restgas nunmehr hauptsächlich aus Alkanen und anderen brennbaren Bestandteilen. Daher ist es sehr gut zur Unterfeuerung der Öfen (Koksofen und/oder Hochofen) geeignet.

In einer Ausgestaltung wird aus dem alkanhaltigen Restgas Methan abgetrennt und als Einsatz in eine Gasturbine zur Erzeugung elektrischer Energie geführt wird. Diese Ausgestaltung der Erfindung kombiniert die Erfindung mit dem Stand der Technik beim dem das Abgas hauptsächlich zur Erzeugung elektrischer Energie verwendet wird. Methan eignet sich von den Bestandteilen des Abgases am besten zur Verwendung in einer Gasturbine zur Erzeugung von elektrischer Energie und wird in dieser Ausgestaltung der Erfindung vom alkanhaltigen Restgas abgetrennt und als Einsatz in eine Gasturbine geführt oder in ein vorhandenes Erdgasnetz eingespeist.

In einer alternativen Ausgestaltung der Erfindung wird nach dem Verfahren zur Bildung von Dimethylether eine Fraktion aus DME-haltigen Produktgas abgetrennt, die Kohlenwasserstoffe mit höchstens einem Kohlenstoffatom enthält. Diese Fraktion besteht in dieser Ausgestaltung der Erfindung im Wesentlichen aus Methan. Zweckmäßigerweise wird Wasserstoff mittels kryogener Trennverfahren aus dem olefinhaltigen Produktgas abgetrennt. Falls das olefinhaltige Produktgas noch Wasserstoff enthält, der in den vorhergehenden Verfahrensschritten nicht umgesetzt wurde, wird dieser mit der kryogenen Trennsequenz (beispielsweise bei Einspeisung des olefinhaltigen Produktgases in eine vorhandene Olefinanlage aber auch bei separater Trennsequenz) automatisch abgetrennt und kann als Produkt anderweitig in der Anlage verwendet oder ausgeführt werden.

In einer weiteren Ausgestaltung der Erfindung wird das alkanhaltige Restgas in ein Verfahren zur partiellen Oxidation von Alkanen in Alkene und Alkine unter Anwesenheit von Sauerstoff geführt, wodurch ein Oxidations-Produktgas entsteht, welcher in das Trennverfahren zur Abtrennung der Olefine zurückgeführt wird. Vorteilhafterweise wird der Wasserstoff und das Oxidations-Produktgas in ein Verfahren zur katalytischen Hydrierung von Alkinen geführt wird, wodurch ein Hydrier-Produktgas entsteht, und das Hydrier-Produktgas in das Trennverfahren zur Abtrennung der Olefine zurückgeführt wird.

Die beschriebenen Rückströme enthalten ebenfalls Olefine, insbesondere Ethylen und/oder Propylen, welche die Ethylen und/oder Propylen Ausbeute und somit die Wirtschaftlichkeit weiter erhöhen.

In einer anderen Ausgestaltung der Erfindung wird das alkanhaltige Restgas in ein thermisches Verfahren unter Sauerstoffabwesenheit geführt wird, wodurch ein Pyrolyse-Produktgas und Kohlenstoff entsteht, und wobei das Pyrolyse-Produktgas in ein Druckwechsel-Absorptionsverfahren geführt wird, wo es in Wasserstoff und ein acetylenhaltiges Restgas zerlegt wird. Das acetylenhaltige Restgas besteht nahezu überwiegend aus Acetylen, welches als Wertprodukt ausgeführt oder als Brennstoff in der Anlage verwendet werden kann. Neben der Verwendung eines Druckwechsel-Absorptionsverfahrens sind auch alternative, dem Fachmann vertraute Verfahren wie Membrantrennverfahren oder, insbesondere bei höhereen Acetylenghalten, auch chemische Wäschen beinhaltend mindestens eine Wasch- und Regenerationsstufe denkbar.

Zweckmäßigerweise wird der Wasserstoff als Wasserstoff-Produkt in anderen Teilen des Stahlwerks, der Kokerei und/oder des Hüttenwerkes und/oder außerhalb dieser Werke verwertet.

In einer weiteren Ausgestaltung der Erfindung wird das Kokereiabgas stromaufwärts des Verfahrens zur Bildung von Dimethylether in ein Verfahren zur Reformierung von Methan unter Bildung von Kohlenmonoxid geführt, wobei ein Reformer-Produktgas entsteht. In dieser Ausgestaltung der Erfindung wird der Kohlenmonoxidanteil im Eingang des Verfahrens zur Bildung von Dimethylether erhöht und somit die Produktbildung dieses Verfahrens begünstigt. Somit können im nachfolgenden Verfahrensschritt mehr Olefine, insbesondere Ethylen und/oder Propylen, gebildet werden. Zusätzlich wird der Methananteil im olefinhaltigen Produktgas geringer und somit die Abtrennung der Olefine, insbesondere Ethylen und/oder Propylen, vereinfacht. In einer alternativen Ausgestaltung kann der Reformers mit einem Wassergas-Shift Reaktor kombiniert werden.

Ebenso kann das alkanhaltige Restgas mit dem Abgas in das Verfahren zur Reformierung von Methan zur Erhöhung des Kohlenmonoxidanteils stromaufwärts des Verfahrens zur Bildung von Dimethylether rückgeführt werden.

In einer alternativen Ausgestaltung der Erfindung wird das Abgas zumindest teilweise stromaufwärts des Verfahrens zur Bildung von Dimethylether und vor der Zumischung von Wasserstoff in ein Verfahren zur Entfernung von Kohlendioxid, Stickstoff und/oder Methan geführt. Diese Ausgestaltung der Erfindung ist besonders für Standorte mit einer geringen Menge an günstig verfügbaren Wasserstoff geeignet. Diese Ausgestaltung der Erfindung ist insbesondere an Standorten mit geringer Menge günstigen Wasserstoffs vorteilhaft. In dieser Ausgestaltung der Erfindung werden potentielle Wasserstoffverbraucher der nachfolgenden Verfahrensschritte entfernt. So reagiert beispielsweise Kohlendioxid mit Wasserstoff zu Kohlenmonoxid und Wasser bei geeigneten Bedingungen oder Katalysatoren. Durch die Entfernung der Wasserstoffkonsumenten in dieser Ausgestaltung der Erfindung wird ein relativ reines Synthesegas aus Kohlenmonoxid und Wasserstoff in das Verfahren zur Bildung von Dimethylether und die nachfolgenden Verfahrensschritte geführt.

Mit der vorliegenden Erfindung gelingt es insbesondere, ein alternatives Verfahren der eingangs erwähnten Art zur Verfügung zu stellen. Die Belastung der Umwelt durch Kohlenmonoxid oder Kohlendioxid aus Hütten- und/oder Stahlwerken zu minimieren, wobei gleichzeitig aus den Abgasen Wertprodukte wie Ethylen und/oder Propylen gewonnen werden. Die Wirtschaftlichkeit des Betriebs von Hütten- und/oder Stahlwerken wird daher durch die vorliegende Erfindung erhöht.

Im Folgenden soll die Erfindung anhand der in den Figuren dargestellten Ausführungsbeispiele näher erläutert werden. Die in den Figuren dargestellten Verfahrensschemata beschreiben jeweils das erfindungsgemäße Verfahren eines integrierten Stahlwerkes mit Koksofen, Hochofen und Konverterofen. Dabei wurden gleiche Teile/Verfahrensschritte mit gleichen Bezugsziffern bezeichnet.

Es zeigen

Figur 1   ein Ausführungsbeispiel mit Methan Reformierung,
Figur 2   ein Ausführungsbeispiel ohne Methan Reformierung,
Figur 3   ein Ausführungsbeispiel mit partieller Oxidation des alkanhaltigen Restgases und
Figur 4   ein Ausführungsbeispiel mit sauerstofffreier Pyrolyse des alkanhaltigen Restgases.

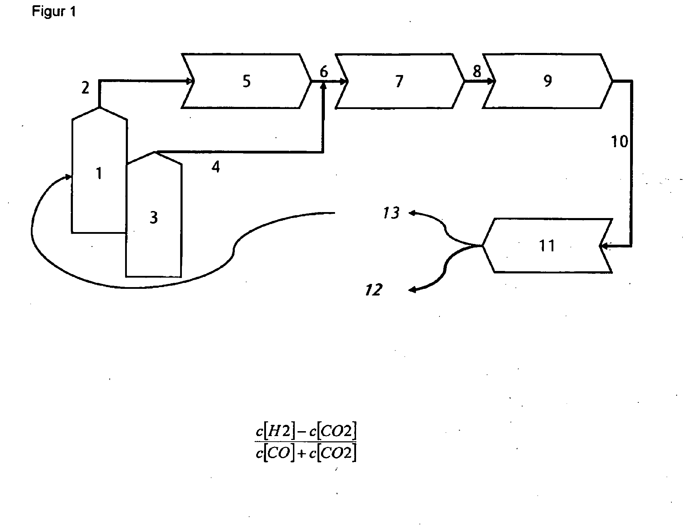

Figur 1 zeigt ein grobes Verfahrensschema eines Ausführungsbeispieles mit Methan Reformierung in einem integrierten Stahlwerk mit Koksofen, Hochofen und Konverterofen.

Im Koksofen 1 wird in Abwesenheit von Sauerstoff Kohle in Koks pyrolisiert. Das dabei entstehende Kokereigas 2 enthält neben dem Hauptbestandteil Wasserstoff Kohlenmonoxid, Kohlendioxid und Methan. Das Kokereigas 2 wird in eine Reformierung 5 zur Umwandlung von Methan in Kohlenmonoxid geführt, wodurch ein Reformer-Produktgas 6 entsteht. Das Reformer-Produktgas 6 ist nahezu Methan frei und hat einen deutlich höheren Anteil Kohlenmonoxid als das Kokereigas 2.

Im Konverterofen 3 wird der Kohlenstoff vom Roheisen entfernt und Stahl erzeugt. Das dabei entstandene Abgas 4 wird mit Reformer-Produktgas 6, ein wasserstoffhaltiges Gas, gemischt und als Einsatz in ein Verfahren 7 zur Bildung von Dimethylether geführt wird, wodurch ein DME-haltiges Produktgas 8 gebildet wird. Durch das Verfahren 7 wird hauptsächlich Kohlenmonoxid und Kohlendioxid über einen Katalysator mit einem oder mehreren aktiven Zentren mit Wasserstoff in Dimethylether umgewandelt.

Das DME-haltige Produktgas 8 wird als Einsatz in ein Verfahren 9 zur Umwandlung von Dimethylether in Olefine, insbesondere Ethylen und/oder Propylen, geführt, wodurch sich ein olefinhaltiges Produktgas 10 bildet. In diesem Verfahren 8 wurden die somit die umweltschädlichen Bestandteile Kohlenmonoxid und Kohlendioxid des Abgases 4 in Wertprodukte 12 verwandelt, die mittel Trennverfahren 11 aus dem olefinhaltigen Produktgas 10 abgetrennt werden. Als Wertprodukte 12 werden insbesondere Ethylen und/oder Propylen gewonnen. Das nach der Abtrennung der Wertprodukte 12 verbliebene alkanhaltige Restgas 13 besteht hauptsächlich aus Alkanen und anderen brennbaren Bestandteilen und wird zur Unterfeuerung in den Koksofen 1 oder den Hochofen (nicht dargestellt) zurückgeführt 14.

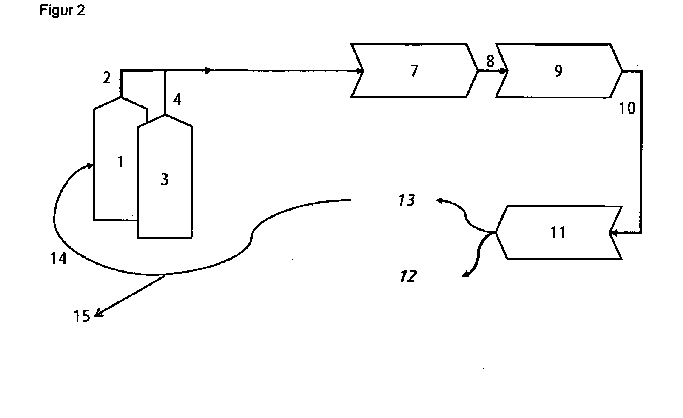

Das in Figur 2 dargestellte Ausführungsbeispiel entspricht dem Beispiel aus Figur 1 jedoch wurde hier auf ein Verfahren zur Methan Reformierung verzichtet. Das Abgas 4 aus dem Konverterofen 3 wird direkt mit dem wasserstoffhaltigen Kokereigas 2 vermischt und als Einsatz in ein Verfahren 5 zur Bildung von Dimethylether geführt. Methan ist bezüglich der folgenden Verfahren inert und wird so in dieser Ausgestaltung der Erfindung mit dem alkanhaltigen Restgas 13 zur Unterfeuerung 14 in den Koksofen 1 und in den Hochofen 15 zurückgeführt.

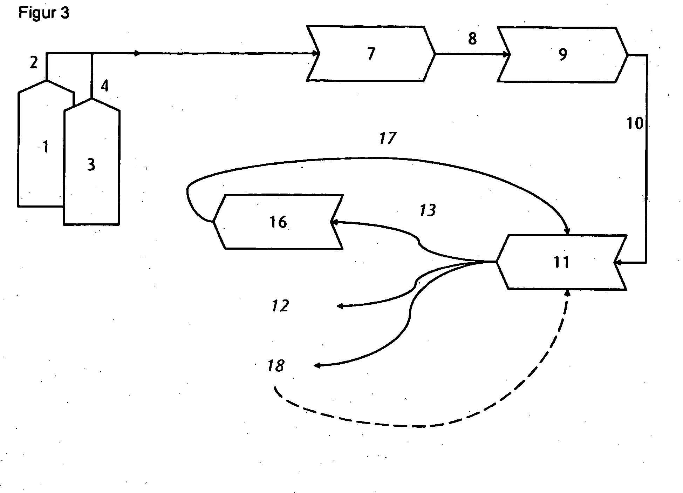

Das in Figur 3 dargestellte Ausführungsbeispiel ähnelt dem Beispiel aus Figur 2, jedoch wird gemäß diesem Ausführungsbeispiel der Erfindung das alkanhaltige Restgas 13 in ein thermisches Verfahren 16 zur Umwandlung von Alkanen in Alkene und Alkine unter Anwesenheit von Sauerstoff geführt wird, wodurch ein Oxidations-Produktgas 17 entsteht, welches in das Trennverfahren 11 zur Abtrennung der Olefine 12 zurückgeführt wird. Im thermischen Pyrolyseverfahren 16 werden bei optionaler Anwesenheit eines Katalysators Alkene und Alkine gebildet. In dieser Ausgestaltung wird in kryogenen Trennverfahren 11 auch Wasserstoff 18 aus dem olefinhaltigen Produktgas 10 abgetrennt. Dieser kann irgendwo in der Anlage verwertet werden oder zur Hydrierung der Alkine des Oxidations-Produktgases 17 in das Trennverfahren 11 zurückgeführt werden (gestrichelte Linie).

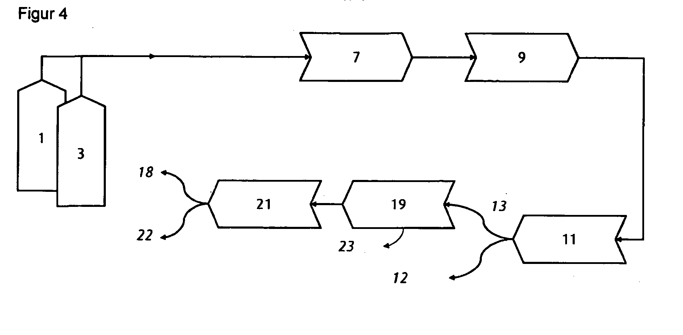

Das in Figur 4 dargestellte Ausführungsbeispiel ähnelt dem Beispiel aus Figur 3 jedoch wird gemäß dieses Ausführungsbeispiel der Erfindung das alkanhaltige Restgas 13 in ein thermisches Verfahren 19 zur Umwandlung von Alkanen in Alkene und Alkine in Abwesenheit von Sauerstoff geführt, wobei in Anwesenheit eines Katalysators hauptsächlich Acetylen und Wasserstoff entstehen. Dieses Pyrolyse-Produktgas 20 wird in ein Verfahren zur Druckwechselabsorption 21 geführt und in die Hauptkomponenten Wasserstoff 18 und Acetylen 22 zerlegt.

Der Wasserstoff 18 kann in beliebigen Anlagenteilen verwendet werden, insbesondere ist die Verwendung als Reduktionsmittel bei der katalytischen Entfernung von Stickoxiden aus Rauchgasen möglich.

**Patentansprüche**

1. Verfahren zur Verarbeitung von Abgas (4) aus einem Stahlwerk und/oder einem Hüttenwerk, wobei das Abgas (4) Kohlendioxid und/oder Kohlenmonoxid enthält und zumindest teilweise zusammen mit einem wasserstoffhaltigen Gas (2,6) in ein Verfahren (7) zur Bildung von Dimethylether geführt wird, wodurch ein DME-haltiges Produktgas (8) gebildet wird, wobei am Eingang des Verfahrens (7) zur Bildung Dimethylether ein Verhältnis von Wasserstoff zu Kohlenmonoxid gewichtet mit der Kohlendioxidkonzentration $\frac{c[H2] - c[CO2]}{c[CO] + c[CO2]}$ von 0.9 bis 1.1 eingestellt wird, wobei das DME-haltige Produktgas (8) in ein Verfahren (9) zur Umwandlung von Diemethylether zu Olefinen geführt wird, wodurch ein olefinhaltiges Produktgas (10) gebildet wird, und wobei mittels Trennverfahren (11) Olefine (12), insbesondere Ethylen und/oder Propylen, aus dem olefinhaltigen Produktgas abgetrennt wird/werden.

2. Verfahren nach Anspruch 1, **dadurch gekennzeichnet, dass** das Abgas (4) aus einem Hochofen und/oder einem Konverterofen und /oder aus einem Direktreduktionsverfahren für Eisenerz abgeführt wird.

3. Verfahren nach Anspruch 1 oder 2, **dadurch gekennzeichnet, dass** Kokereiabgas (2), insbesondere Abgas (2) aus einem Koksofen, als wasserstoffhaltige Gas zusammen mit zumindest einem Teil des Abgases (4) in das Verfahren (7) zur Bildung von Dimethylether geführt wird.

4. Verfahren nach einem der Ansprüche 1 bis 3, **dadurch gekennzeichnet, dass** am Eingang des Verfahrens (7) zur Bildung von Dimethylether ein Verhältnis von Wasserstoff zu Kohlenmonoxid gewichtet mit der Kohlendioxidkonzentration $\frac{c[H2] - c[CO2]}{c[CO] + c[CO2]}$ von 1 eingestellt wird.

5. Verfahren nach einem der Ansprüche 1 bis 4, **dadurch gekennzeichnet, dass** das olefinhaltige Produktgas (10) nach Abtrennung der Olefine (12) als alkanhaltiges Restgas (13) zur Unterfeuerung in den Koksofen und/oder Hochofen zurückgeführt (14) wird.

**6.** Verfahren nach einem der Ansprüche 1 bis 5, **dadurch gekennzeichnet, dass** aus dem alkanhaltigen Restgas (13) Methan (15) abgetrennt und als Einsatz in eine Gasturbine zur Erzeugung elektrischer Energie geführt wird.

**7.** Verfahren nach einem der Ansprüche 1 bis 6, **dadurch gekennzeichnet, dass** Wasserstoff (18) mittels kryogener Trennverfahren (11) aus dem olefinhaltigen Produktgas abgetrennt wird.

**8.** Verfahren nach einem der Ansprüche 1 bis 7, **dadurch gekennzeichnet, dass** das alkanhaltige Restgas (13) in ein Verfahren zur partiellen Oxidation (16) von Alkanen in Alkene und Alkine unter Anwesenheit von Sauerstoff geführt wird, wodurch ein Oxidations-Produktgas (17) entsteht, und das Oxidations-Produktgas (17) in das Trennverfahren (11) zur Abtrennung der Olefine (12) zurückgeführt wird.

**9.** Verfahren nach Anspruch 8, **dadurch gekennzeichnet, dass** der Wasserstoff (18) und das Oxidations-Produktgas (17) in ein Verfahren zur katalytischen Hydrierung von Alkinen geführt wird, wodurch ein Hydrier-Produktgas entsteht, und das Hydrier-Produktgas in das Trennverfahren (11) zur Abtrennung der Olefine zurückgeführt wird.

**10.** Verfahren nach einem der Ansprüche 1 bis 7, **dadurch gekennzeichnet, dass** alkanhaltiges Restgas (13) in ein thermisches Verfahren (19) unter Sauerstoffabwesenheit geführt wird, wodurch ein Pyrolyse-Produktgas und Kohlenstoff (23) entsteht, und wobei das Pyrolyse-Produktgas in ein Druckwechsel-Absorptionsverfahren (21) geführt wird, wo es in Wasserstoff (18) und ein acetylenhaltiges Restgas (22) zerlegt wird.

**11.** Verfahren nach einem der Ansprüche 7 bis 10, **dadurch gekennzeichnet, dass** der Wasserstoff (18) als Wasserstoff-Produkt in anderen Teilen des Stahlwerks, der Kokerei und/oder des Hüttenwerkes und/oder außerhalb dieser Werke verwertet wird.

**12.** Verfahren nach einem der Ansprüche 3 bis 11, **dadurch gekennzeichnet, dass** Kokereiabgas (2) stromaufwärts des Verfahrens zur Bildung von Dimethylether in ein Verfahren (5) zur Reformierung von Methan unter Bildung von Kohlenmonoxid geführt wird, wobei ein Reformer-Produktgas (6) entsteht.

**13.** Verfahren nach einem der Ansprüche 1 bis 12, **dadurch gekennzeichnet, dass** das Abgas (4) stromaufwärts des Verfahrens (7) zur Bildung von Dimethylether in ein Verfahren zur Entfernung von Kohlendioxid, Stickstoff und/oder Methan geführt wird.

## Claims

**1.** Process for processing offgas (4) from a steelworks and/or a smelting works, wherein the offgas (4) contains carbon dioxide and/or carbon monoxide and is fed, at least partly together with a hydrogen-containing gas (2, 6) to a process (7) for forming dimethyl ether, as a result of which a DME-containing product gas (8) is formed, a ratio of hydrogen to carbon monoxide weighted by the carbon dioxide concentration $\frac{c[H2] - c[CO2]}{c[CO] + c[CO2]}$ of from 0.9 to 1.1 is set at the inlet of the process (7) for forming dimethyl ether, the DME-containing product gas (8) is fed to a process (9) for converting dimethyl ether into olefins, as a result of which an olefin-containing product gas (10) is formed, and olefins (12), in particular ethylene and/or propylene, are separated off from the olefin-containing product gas by means of a separation process (11).

**2.** Process according to Claim 1, **characterized in that** the offgas (4) is discharged from a blast furnace and/or a converter and/or from a direct reduction process for iron ore.

**3.** Process according to Claim 1 or 2, **characterized in that** coking plant offgas (2), in particular offgas (2) from a coke oven, is fed as hydrogen-containing gas together with at least a part of the offgas (4) into the process (7) for forming dimethyl ether.

**4.** Process according to any of Claims 1 to 3, **characterized in that** a ratio of hydrogen to carbon monoxide weighted by the carbon dioxide concentration $\frac{c[H2] - c[CO2]}{c[CO] + c[CO2]}$ of 1 is set at the inlet of the process (7) for forming dimethyl ether.

5. Process according to any of Claims 1 to 4, **characterized in that** the olefin-containing product gas (10) is, after separating off the olefins (12), recirculated (14) as alkane-containing tailgas (13) for bottom firing to the coke oven and/or blast furnace.

6. Process according to any of Claims 1 to 5, **characterized in that** methane (15) is separated off from the alkane-containing tailgas (13) and fed as feed into a gas turbine for generating electric energy.

7. Process according to any of Claims 1 to 6, **characterized in that** hydrogen (18) is separated off from the olefin-containing product gas by means of a cryogenic separation process (11).

8. Process according to any of Claims 1 to 7, **characterized in that** the alkane-containing tailgas (13) is fed to a process for the partial oxidation (16) of alkanes to alkenes and alkynes in the presence of oxygen, resulting in formation of an oxidation product gas (17), and the oxidation product gas (17) is recirculated to the separation process (11) for separating off the olefins (12).

9. Process according to Claim 8, **characterized in that** the hydrogen (18) and the oxidation product gas (17) are fed to a process for the catalytic hydrogenation of alkynes, as a result of which a hydrogenation product gas is formed, and the hydrogenation product gas is recirculated to the separation process (11) for separating off the olefins.

10. Process according to any of Claims 1 to 7, **characterized in that** alkane-containing tailgas (13) is fed to a thermal process (19) in the absence of oxygen, as a result of which a pyrolysis product gas and carbon (23) are formed, and the pyrolysis product gas is fed to a pressure swing absorption process (21) where it is separated into hydrogen (18) and an acetylene-containing tailgas (22).

11. Process according to any of Claims 7 to 10, **characterized in that** the hydrogen (18) is utilized as hydrogen product in other parts of the steelworks, the coking plant and/or the smelting works and/or outside these works.

12. Process according to any of Claims 3 to 11, **characterized in that** coking plant offgas (2) is fed into a process (5) for reforming methane to form carbon monoxide upstream of the process for forming dimethyl ether, forming a reformer product gas (6).

13. Process according to any of Claims 1 to 12, **characterized in that** the offgas (4) is fed into a process for removing carbon dioxide, nitrogen and/or methane upstream of the process (7) for forming dimethyl ether.


**Revendications**

1. Procédé de traitement d'un effluent gazeux (4) d'une aciérie et/ou d'une usine sidérurgique, l'effluent gazeux (4) contenant du dioxyde de carbone et/ou du monoxyde de carbone, et étant introduit au moins en partie conjointement avec un gaz contenant de l'hydrogène (2, 6) dans un procédé (7) de formation d'éther diméthylique, par lequel un courant gazeux de produits contenant du DME (8) est formé, un rapport entre l'hydrogène et le monoxyde de carbone pondéré par la concentration en dioxyde de carbone $\dfrac{c[H2]-c[CO2]}{c[CO]+c[CO2]}$ de 0,9 à 1,1 étant ajusté à l'entrée du procédé (7) de formation d'éther diméthylique, le courant gazeux de produit contenant du DME (8) étant introduit dans un procédé (9) de conversion de l'éther diméthylique en oléfines, par lequel un courant gazeux de produits contenant des oléfines (10) est formé, et des oléfines (12), notamment de l'éthylène et/ou du propylène, étant séparées du courant gazeux de produits contenant des oléfines par des procédés de séparation (11).

2. Procédé selon la revendication 1, **caractérisé en ce que** l'effluent gazeux (4) est déchargé d'un haut fourneau et/ou d'un four de conversion et/ou d'un procédé de réduction directe pour minerai de fer.

3. Procédé selon la revendication 1 ou 2, **caractérisé en ce qu'**un effluent gazeux de cokerie (2), notamment un effluent gazeux (2) d'un four à coke, est introduit dans le procédé (7) pour la formation d'éther diméthylique en tant que gaz contenant de l'hydrogène conjointement avec au moins une partie de l'effluent gazeux (4).

**4.** Procédé selon l'une quelconque des revendications 1 à 3, **caractérisé en ce qu'**un rapport entre l'hydrogène et le monoxyde de carbone pondéré par la concentration en dioxyde de carbone $\dfrac{c[H2]-c[CO2]}{c[CO]+c[CO2]}$ de 1 est ajusté à l'entrée du procédé (7) de formation d'éther diméthylique.

**5.** Procédé selon l'une quelconque des revendications 1 à 4, **caractérisé en ce que** le courant gazeux de produits contenant des oléfines (10) est recyclé sous forme d'un gaz résiduel contenant des alcanes (13) après la séparation des oléfines (12) dans le four à coke et/ou le haut fourneau pour le chauffage.

**6.** Procédé selon l'une quelconque des revendications 1 à 5, **caractérisé en ce que** du méthane (15) est séparé du gaz résiduel contenant des alcanes (13) et est introduit en tant que charge dans une turbine à gaz pour la génération d'énergie électrique.

**7.** Procédé selon l'une quelconque des revendications 1 à 6, **caractérisé en ce que** de l'hydrogène (18) est séparé du courant gazeux de produits contenant des oléfines par des procédés de séparation cryogéniques (11).

**8.** Procédé selon l'une quelconque des revendications 1 à 7, **caractérisé en ce que** le gaz résiduel contenant des alcanes (13) est introduit dans un procédé d'oxydation partielle (16) d'alcanes en alcènes et alcynes en présence d'oxygène, par lequel un courant gazeux de produits d'oxydation (17) est formé, et le courant gazeux de produits d'oxydation (17) est recyclé dans le procédé de séparation (11) pour la séparation des oléfines (12).

**9.** Procédé selon la revendication 8, **caractérisé en ce que** l'hydrogène (18) et le courant gazeux de produits d'oxydation (17) sont introduits dans un procédé d'hydrogénation catalytique d'alcynes, par lequel un courant gazeux de produits d'hydrogénation est formé, et le courant gazeux de produits d'hydrogénation est recyclé dans le procédé de séparation (11) pour la séparation des oléfines.

**10.** Procédé selon l'une quelconque des revendications 1 à 7, **caractérisé en ce que** le gaz résiduel contenant des alcanes (13) est introduit dans un procédé thermique (19) en l'absence d'oxygène, par lequel un courant gazeux de produits de pyrolyse et du carbone (23) sont formés, et le courant gazeux de produits de pyrolyse est introduit dans un procédé d'absorption modulée en pression (21), où il est décomposé en hydrogène (18) et un gaz résiduel contenant de l'acétylène (22).

**11.** Procédé selon l'une quelconque des revendications 7 à 10, **caractérisé en ce que** l'hydrogène (18) est utilisé en tant que produit hydrogène dans d'autres parties de l'aciérie, de la cokerie et/ou de l'usine sidérurgique et/ou en dehors de ces usines.

**12.** Procédé selon l'une quelconque des revendications 3 à 11, **caractérisé en ce que** l'effluent gazeux de cokerie (2) est introduit en amont du procédé de formation d'éther diméthylique dans un procédé (5) de reformage de méthane avec formation de monoxyde de carbone, un courant gazeux de produits de reformeur (6) se formant.

**13.** Procédé selon l'une quelconque des revendications 1 à 12, **caractérisé en ce que** l'effluent gazeux (4) est introduit en amont du procédé (7) de formation d'éther diméthylique dans un procédé d'élimination de dioxyde de carbone, d'azote et/ou de méthane.

Figur 1

$$\frac{c[H2] - c[CO2]}{c[CO] + c[CO2]}$$

Figur 2

Figur 3

Figur 4

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20110041517 A
- WO 2009023987 A
- CN 101913558
- CN 101823937
- CN 102079689
- US 20110112314 A

**In der Beschreibung aufgeführte Nicht-Patentliteratur**

- Ethylene. **H. ZIMMERMANN ; R. WALZL.** Ullmann's Encyclopedia of Industrial Chemistry. Wiley, 2011